# EUROPEAN PATENT SPECIFICATION

(11) **EP 2 992 811 B1**
(45) Date of publication and mention of the grant of the patent: **07.03.2018**
(21) Application number: 15178988.0
(22) Date of filing: 20.04.2006
(51) Int. Cl.: A61B 1/313, A61B 1/00, A61B 90/00, A61B 34/00, A61B 17/00, A61B 17/34, A61B 34/30

(54) **MEANS AND METHODS OF IMPROVING LAPAROSCOPIC SURGERY**
MITTEL UND VERFAHREN ZUR VERBESSERUNG DER LAPAROSKOPISCHEN CHIRURGIE
MOYEN ET PROCEDES PERMETTANT D'AMELIORER LA CHIRURGIE LAPAROSCOPIQUE

(30) Priority: 18.04.2005 US 672010 P; 04.08.2005 US 705199 P; 15.09.2005 US 716951 P; 15.09.2005 US 716953 P
(43) Date of publication of application: 09.03.2016
(62) Divisional of application: 06728279.8
(73) Proprietor: M.S.T. Medical Surgery Technologies Ltd., 20692 Yoqneam (IL)
(72) Inventor: Sholev, Mordehai, 37830 Amikam (IL)
(74) Representative: Lecomte & Partners

(56) References cited:
- US-A- 5 279 309
- US-A- 5 749 362
- US-A1- 2002 156 466
- US-A1- 2004 015 053
- US-A1- 2004 204 627
- US-B1- 6 714 841

## Description

### FIELD OF THE INVENTION

The present invention generally relates to means for improving the interface between the surgeon and an endoscope system for laparoscopic surgery. Moreover, this present invention discloses a camera holder mechanism for laparoscopic surgery and/or a device useful controlling an endoscope system for laparoscopic surgery, in which the endoscope is inserted through a small incision in the abdomen or chest.

### BACKGROUND OF THE INVENTION

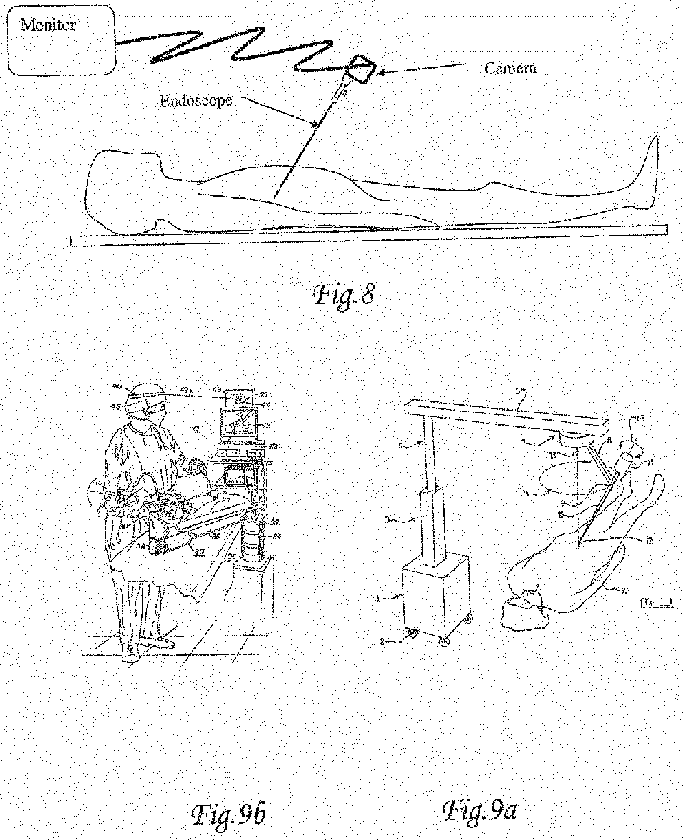

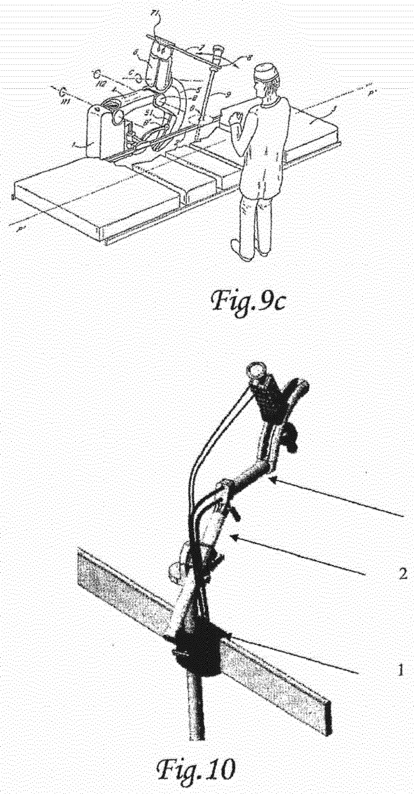

In laparoscopic surgery, the surgeon performs the operation through small holes using long instruments and observing the internal anatomy with an endoscope camera. The endoscope is conventionally held by a camera assistant since the surgeon must perform the operation using both hands. The surgeon performance is largely dependent on the camera position relative to the instruments and on a stable image shown at the monitor. The main problem is the difficulty for the assistant to hold the endoscope steadily, keeping the scene upright. To overcome these problems, several new technologies have been developed, using robotics to hold the endoscope while the surgeon performs the procedure, e.g., Lapman, Endoassist etc. But these technologies are expensive, difficultly installed, uncomfortable to use, limiting the dexterity of the surgeon and having physical dimension much bigger that all operating tools. Relatively to the required action, they also move in big bounds with several arms movement. Reference is made now to figures 9a, b, c, presenting a schematic illustration of the U.S. patents which describes these technologies.

Moreover, radiological e.g. X-ray and ultrasound examinations are able to diagnose many conditions within the abdomen and pelvis but do have their limitations. Under certain circumstances a clearer picture of the appearance of the abdomen and pelvis is required.

The laparoscope is a sterile surgical instrument that has special optics that allows small amounts of light to be transmitted effectively. Carbon dioxide gas is pumped through a channel in the laparoscope into the abdomen. This creates a space within which the surgeon can look or operate. A laparoscopy is performed under a general anesthetic. A small cut measuring 1 to 2cm is made in or just below the belly-button. Through this cut, the laparoscope is gently introduced into the abdomen. Additional surgical instruments are often required. These are usually introduced via even smaller cuts in the skin above or to the side of the pubic hair. Most laparoscopics are performed as part of the investigation of abdominal or pelvic pain. The most common illnesses diagnosed through laparoscopy are endometriosis, pelvic inflammatory disease, ectopic pregnancy, ovarian cysts and appendicitis. In many cases it is possible to perform operations through the laparoscope itself. Most sterilizations today are performed through a laparoscope. Cysts on the ovaries can be punctured and opened, while adhesions caused by ovarian diseases or other diseases of the pelvic organs can also be loosened. Most ectopic pregnancies can be treated by laparoscopic means as can many cases of endometriosis. Laparoscopic surgery is becoming increasingly popular with patients because the scars are smaller and their period of recovery is shorter. Laparoscopic surgery requires special training of the surgeon or gynecologist and the theatre nursing staff. The equipment is often expensive and not available in all hospitals.

During laparoscopic surgery it is often required to shift the spatial placement of the endoscope in order to present the surgeon with the optimal view. Conventional laparoscopic surgery makes use of either human assistants that manually shift the instrumentation or alternatively robotic automated assistants. Automated assistants utilize interfaces that enable the surgeon to direct the mechanical movement of the assistant, achieving a shift in the camera view. US patent no. 5279309 relates to a system for use in laparoscopy comprising an endoscope moved by a computer to achieve a desired relationship with a surgical instrument having beacons connected thereto. US patent 6714841 discloses an automated camera endoscope in which the surgeon is fitted with a head mounted light source that transmits the head movements to a sensor, forming an interface that converts the movements to directions for the mechanical movement of the automated assistant. Alternative automated assistants incorporate a voice operated interface, a directional key interface, or other navigational interfaces. The above interfaces share the following drawbacks:
a. Single directional interface that provide limited feedback to the surgeon
b. Cumbersome serial operation for starting and stopping movement directions that requires the surgeon's constant attention.

Research has suggested that these systems divert the surgeons focus from the major task at hand. Therefore technologies assisted by magnets and image processing have been developed to simplify interfacing control. However these improved technologies still fail to address another complicating interface aspect of laparoscopic surgery, they do not allow the surgeon to signal to both the automated assistant and to surgical colleagues, which instrument his attention is focused on.

### SUMMARY OF THE INVENTION

It is thus one object of the present invention to provide means for improving the interface between the surgeon and an endoscope system for laparoscopic surgery, holding a laparoscopic camera and/or controlling automated endoscope assistant.

It is also in the scope of the present invention a device according to claim 1.

Further developments of the invention are according to dependent claims 2 to 12.

### BRIEF DESCRIPTION OF THE FIGURES

In order to understand the invention and to see how it may be implemented in practice, and by way of non-limiting example only, with reference to the accompanying drawing, in which
FIG. 1 is a general schematic view of an enhanced interface laparoscopic system that relies on a single wireless code signal to indicate the instrument on which to focus the endoscope constructed in accordance with the principles of the present invention in a preferred embodiment thereof;
FIG. 2 is a general schematic view of an enhanced interface laparoscopic system that relies on at least two wireless signals to indicate the instrument on which to focus the endoscope;
FIG. 3 is a schematic view of the method in which the single wireless code signal choice instrumentation focus is represented on the viewing apparatus;
FIG. 4 is a schematic view of the method in which multiple wireless code signal choice of instrumentation is operated;
FIG.5 a is a schematic view of a wireless system;
FIG.5b represents the relative position of each tool in respect to the mechanism;
FIG.5c is a schematic view of the mechanism of the enhanced interface laparoscopic system;
FIG.5d is a schematic cut view of the first part of the mechanism of the enhanced interface laparoscopic system;
FIG.5e, 5f, represent schematic different views of the entire mechanism of the enhanced interface laparoscopic system;
FIG.5g schematically illustrates a mechanism with only one curved guide;
FIG.5h schematically illustrates the four degrees of freedom of the mechanism;
FIG.6a is a schematic view of the second part of the mechanism of the enhanced interface laparoscopic system;
FIG.6b is a schematic view of the telescopic guide;
FIG. 7a, b, c schematically present an illustrating example of a camera holder mechanism for laparoscopic surgery;
FIG. 8 illustrates the way in which the endoscope is inserted through a small incision in the abdomen or chest;
FIG. 9a, b, c present a schematic illustration of U.S Pat 6,714,841 which describes prior art technologies;
FIG. 10 presents a schematic and illustrated drawing of the entire system according to one embodiment of the present invention which comprises three main parts a manipulating endoscope mechanism (1); a force carriage system (2); and a manipulating system actuator (3);
FIG. 11 presents a schematic illustration of the endoscope system according to one embodiment of the present invention;
FIG. 12 is a schematic view of the endoscope system illustrating the motion of the orientation ring relatively to the basis ring;
FIG. 13 is a schematic view of the zoom mechanism according to one embodiment of the present invention;
FIG. 14 is a schematic view of the orientation ring different position;
FIG. 15 presents a schematic description of the rotation mechanism;
FIG. 16 represents the portable feature of the mechanism;
FIG. 17 is a schematic view of the mechanism placed beside a bed;
FIG. 18a, b, 19a, b, 20 represent three different options for the zoom mechanism: 18 a and b with parallelogram rods, 19 a and b with a ring zoom and 20 a and b with a reduction force device;
FIG. 21 presenting a schematic section view of the pulley blocks located on the endoscope motion mechanism;
FIG. 22 is a three-dimension schematic view of figure 21;
FIG. 23 is a schematic view of the zoom mechanism obtained by rotating cable which turns a central screw with joins in different directions;
FIG. 24 presents a schematic and illustrated drawing of the entire system according to one embodiment of the present invention which comprises three main parts a manipulating endoscope mechanism (1); a force carriage system (2); and a manipulating system actuator (3);
FIG. 25 presents a schematic illustration of the manipulating endoscope mechanism (i);
FIG. 26 presents a schematic cut view along the sliding links 1 la, b, c of figure 25;
FIG. 27 presents the zoom mechanism, according to another embodiment of the present invention;
FIG. 28a schematically presents the principle mechanism that controls the linear movement of the endoscope according to another embodiment of the present invention;
FIG. 28b, c schematically present the rotation mechanism according to another embodiment of the present invention;
FIG. 29 schematically presents the envelope of the endoscope range of movement;
FIG. 30 schematically presents the way the mechanism acts to controls one angle of the endoscope by changing the total length of the telescopic arm;
FIG. 31 schematically presents the way the mechanism acts to controls another angle (β) of the endoscope by rotating telescopic arm;
FIG. 32a schematically presents the portable feature of the mechanism; and,
FIG. 32b schematically presents an upper view of the position abilities of the system: the rotation angle γ, and the horizontal position X slider.

### DETAILED DESCRIPTION OF THE EMBODIMENTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide means for improving the interface between the surgeon and an endoscope system for laparoscopic surgery. Moreover, this present invention discloses a camera holder mechanism for laparoscopic surgery and/or a device useful controlling an endoscope system for laparoscopic surgery, in which the endoscope is inserted through a small incision in the abdomen or chest.

The present invention can be also utilized to improve upon the interface between surgeon and mechanical and human assistants by communicating the surgeon's current instrument of choice thereby directing the endoscope to focus on the choice. The technology relies on marrying a conventional laparoscopic system with data obtained button operated small wireless transmitters. In preferred embodiment of the invention a single wireless emission code is utilized and choice is achieved by a visible graphic representation upon the conventional viewing screen. In another preferred embodiment each instrument is fitted with a unique code wireless transmitter, and selection is achieved by depressing its button. The present invention discloses also an interface device to be married with conventional camera assisted laparoscopic surgery systems comprising at least one wireless transmitter that may or may not be attached to the maneuvering control end of surgical instruments. Upon depression of at least one button on the transmitters either a generic or a unique code is transmitted to a receiving device connected to a computer that presents the selection on a connected video screen. Confirmation of the selection by the depression of at least one button on wireless transmitter transmits a code to the receiver connected to the computer that instructs the automated surgical assistant to move the endoscope achieving a view on the screen that is focused on the selected instrument area.

It would thus be desirable to achieve a device that allows the surgeon to identify to the laparoscopic computing system as well as to surgical colleagues to which surgical instrument attention is to be directed thereby directing the view achieved by the endoscope to the selected focus of attention.

Therefore, in accordance with a preferred embodiment of the present invention, there is provided an enhanced interface laparoscopy device comprising:
a. At least one wireless transmitter with at least one operating key.
b. At least one wireless receiver.
c. At least one conventional laparoscopy computerized system loaded with conventional surgical instrument spatial location software, and conventional automated assistant maneuvering software.
d. Software loaded onto to the conventional laparoscopy system that enables a visual response to the depression of at least one key on the wireless transmitter as well as an interface with the conventional automated assistant maneuvering software so as to achieve movement of the endoscope.
e. At least one video screen.
f. At least one automated assistant.

In a preferred embodiment of the enhanced interface laparoscopy device the wireless transmitter or transmitters are either freestanding or attached to the maneuvering end of the surgical instruments and emit the same single code that upon the depression of at least one key on them emits a signal to the receiver that communicates with the connected computer that superimposes a graphic symbol upon a random choice of one of the onscreen surgical instruments depicted by the computer on the connected computer screen. The surgeon repeats the depression of at least one key resulting in a shift in the superimposed graphic designator from one onscreen depiction of surgical instrument to another until the desired instrument is reached and thereby selected. Subsequently the computer directs the automated assistant to focus the endoscope on the desired instrument area. In a further preferred embodiment the selection of the instrument requires confirmation by varying the form of click on at least one key, such as a prolonged depression. Only upon confirmation is the computer authorized to instruct the automated assistant to focus the endoscope on the desired instrument area. In another preferred embodiment of the invention each relevant surgical instruments is fitted at its maneuvering control end with a wireless transmitter with at least one key that transmits a unique code. In the initial stage of the procedure the surgeon identifies each of the instruments to the computerized system by depressing at least one key on each of the wireless transmitters fitted to the surgical instruments and matching their characteristics with a prepared database, thereby forming within the computerized system a unique signature for each of the transmitters. Thereon, upon depression of at least one key on the wireless transmitter attached to each surgical instrument, the receiver receives the unique code communicates it to the computer that identifies it with the preprogrammed signature and instructs the automated assistant to move the endoscope so as to achieve the desired focus. In a further preferred embodiment the selection is signified on the connected screen by superimposing a graphic symbol upon the onscreen depiction of the surgical. In a further preferred embodiment the selection is confirmed by an additional mode of depression of at least one key on the wireless transmitter, such as a prolonged depression of the key, authorizing the computer to instruct the automated assistant to change view provided by the endoscope.

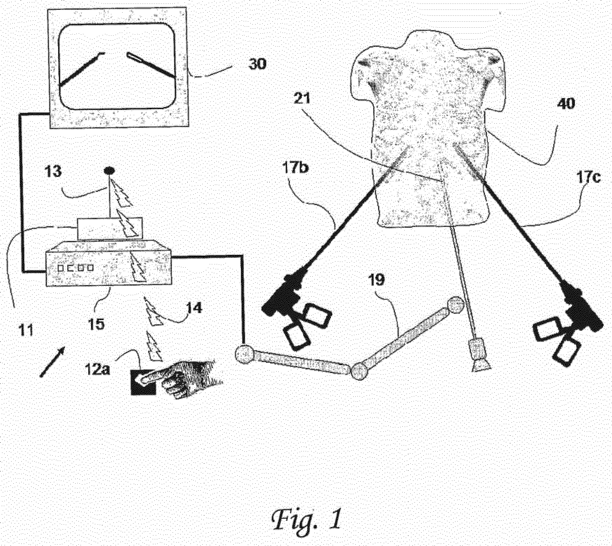

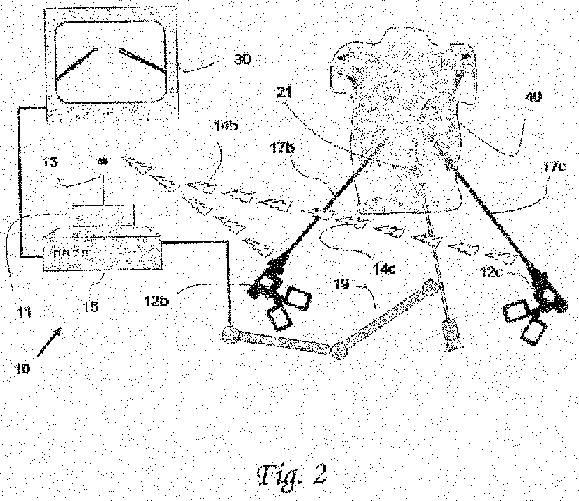

The device of the present invention has many technological advantages, among them:
- Simplifying the communication interface between surgeon and mechanical assistants.
- Seamless interaction with conventional computerized automated endoscope systems.
- Simplicity of construction and reliability.
- User-friendliness
- Additional features and advantages of the invention will become apparent from the following drawings and description.
Reference is made now to figure 1, which is a general schematic view of an enhanced interface laparoscopic system comprising one or more button operated wireless transmitters 12a, that may or may not be attached to the maneuvering end of surgical instruments 17b and 17c, which once depressed aerially transmit a single code wave 14 through aerial 13 to connected receiver 11 that produces a signal processed by computer 15 thereby assigning a particular one of two or more surgical instruments 17b and 17c as the focus of the surgeons attention. Accordingly a conventional automated endoscope 21 is maneuvered by means of conventional automated arm 19 according to conventional computational spatial placement software contained in computer 15.
Reference is made now to figure 2, which is a is a general schematic view of an enhanced interface laparoscopic system comprising one or more button operated wireless transmitters 12b and 12c are attached respectfully to the maneuvering means at the end of surgical instruments 17b and 17c, which once depressed aerially, each transmit a unique code wave 14b and 14c through aerial 13 to connected receiver 11 that produces a signal processed by computer 15 thereby assigning a particular one of two or more surgical instruments 17b and 17c as the focus of the surgeons attention. Accordingly a conventional automated endoscope 21 is maneuvered by means of conventional automated arm 19 according to conventional computational spatial placement software contained in computer 15 .

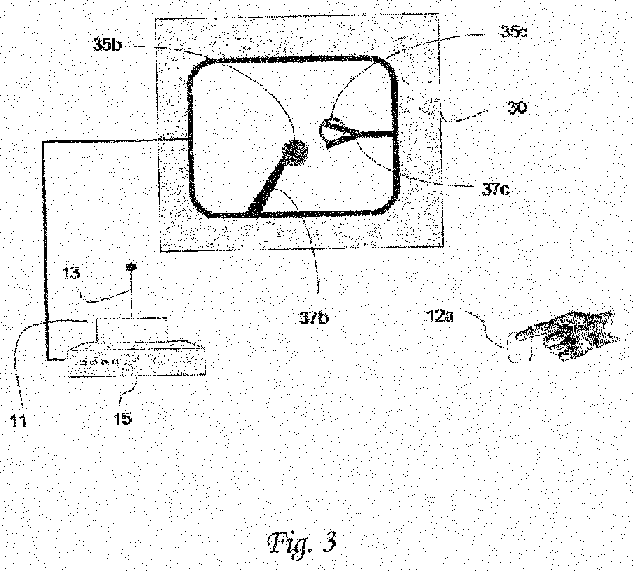

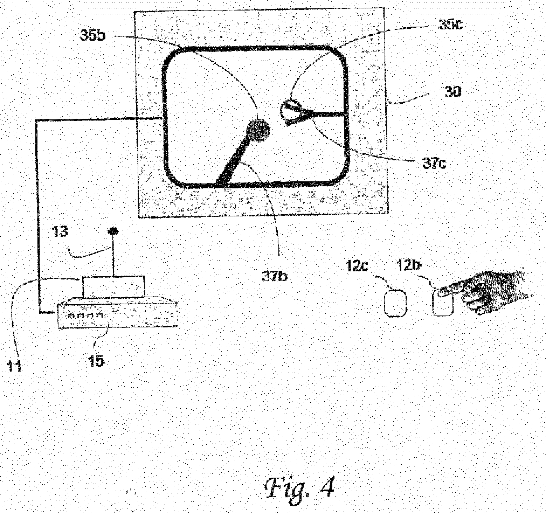

Reference is made now to figure 3, which is a schematic view of the method in which single wireless signal code choice of instrumentation focus is achieved, by means of video representation 37b and 37c of the actual surgical instruments (not represented in fig.3) superimposed by graphic symbols. Wherein a light depression of the button on generic code emitting wireless transmitter 12a transmits a code that is received by receiver aerial 13 communicated through connected receiver 11 to computer 15 that shifts the graphically superimposed symbol of choice 35b on video screen 30 from instrument to instrument until the required instrument is reached. A prolonged depression of the button on transmitter 12a confirms the selection thereby signaling computer 15 to instruct the automated mechanical assistant (not represented in fig. 3) to move the endoscope (not represented in fig.3) and achieving a camera view of the instrument area on screen 30.
Reference is made now to figure 4, which is a schematic view of the method in which multiple wireless signal code choice of instrumentation focus is achieved, by means of video representation 37b and 37c of the actual surgical instruments (not represented in fig.4) superimposed by graphic symbols. Wherein when buttons on unique code emitting wireless transmitters 12b and 12c attached respectfully to actual operational instruments (not represented in fig. 4) superimposes graphic symbol 35b on respectful video representation 37b. A prolonged depression of the button on transmitter 12a confirms the selection thereby signaling computer 15 to instruct the automated mechanical assistant (not represented in fig. 4) o move the endoscope (not represented in fig.4) and achieving a camera view of the instrument area on screen 30.

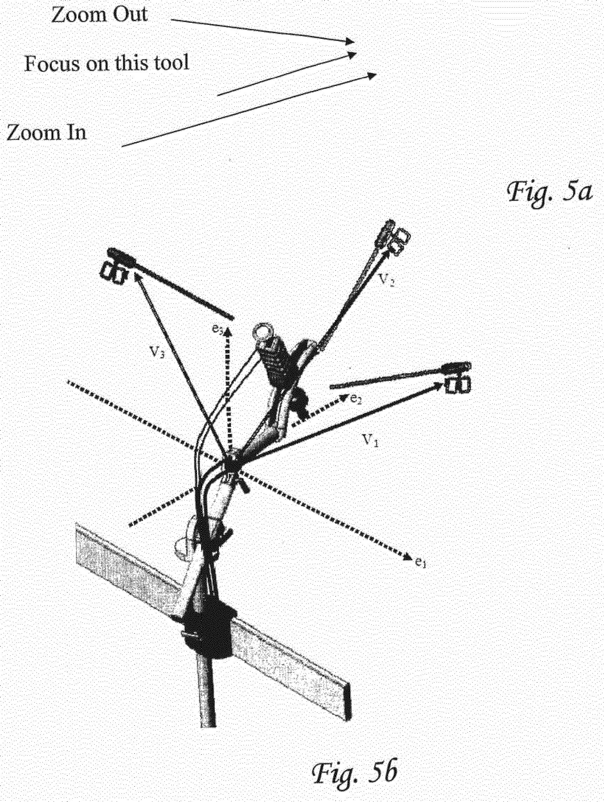

Reference is made now to figure 5a illustrating an example of a wireless system. The wireless device attached to the tool transmits three action codes to the camera holder positioning sensors array: zoom up, zoom down, change position to get better view of a tool.

Reference is made now to figure 5b illustrating the relative position of each tool in respect to the mechanism: while performing the surgery the surgeon often changes the position of his tools and even their insertion point. The wireless switches then may be use to locate the relative angle in which each tool is being held in respect to the camera holder mechanism. This is another advantage of the system that is used to calculate the position of the tool in the frame captured by the video camera. In that manner the surgeon does not have to inform the system where the insertion point of every tool is.The exact location of the wireless switch does not measured: the information about the relative positions of the tools in respect to each other contains in most cases enough data for the software to maintain the matching between the switches and the tools. In this figure the positioning sensors of the system are placed near or on the camera holder so the signals they receive can be utilize in order to calculate the vectors V1 V2 ...Vn representing the range and the 3 angles needed to define a point in a 3D space.
In order to realize a position and range system, many well-known technologies may be used. For example if the switches emit wireless signals then an array of antennas may be used to compare the power of the signal received at each antenna in order to determine the angle of the switch and it's approximate range to the camera holder mechanism. If the switch emits ultra sound wave then US microphones can be used to triangulate the position of the switch. The same is for light emitting switch. At any case this position system does not included in the scope of this invention.

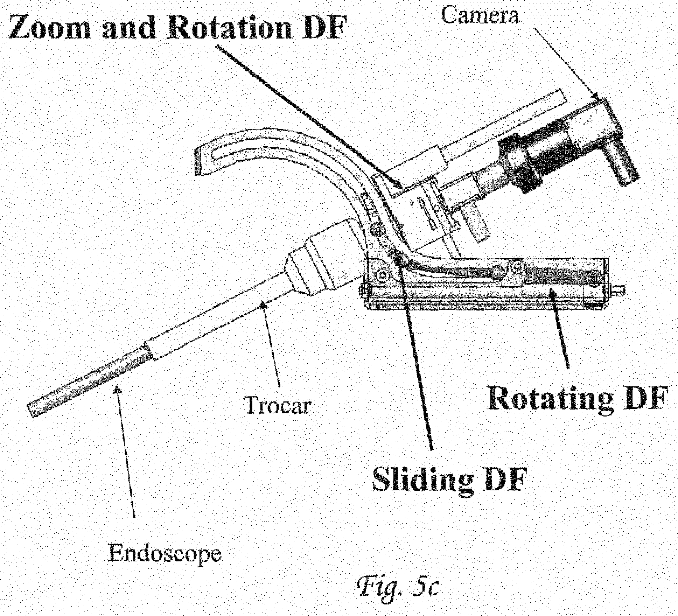

Reference is made now to figure 5c illustrating the mechanism of the system which enables the positioning of an endoscope while performing a laparoscopy surgery. The system consists on two main components: the first part has an arc shape in which the endoscope can be driven back and forth and at the same time can be move from side to side; the second part is characterized by zoom and rotation properties. The mechanism allows the moving and the positioning of the endoscope in the angles of 0°-180° back and forth and O0- 180° side to side. The first consists of arc shape housing which moves a gimbal mechanism along an arc shape guide. The base of the arc includes a housing containing a lead screw that moves a nut back and forth. The moving nut is connected to the gimbal with rigid links that transfer the linear nut movement to the gimbals mechanism resulting its movement back and forth along the arc shape guide. The lead screw housing (back forth screw housing) is connected to another mechanism which rotates the first part from side to side around the long axis of the lead screw. This mechanism also supply the moments needed to rotate the lead screw. This design allows the motors that move the first part to be connected from a distance preferably by flexible shafts. The separation is a very useful feature because at that manner the presence of the mechanism becomes minor.

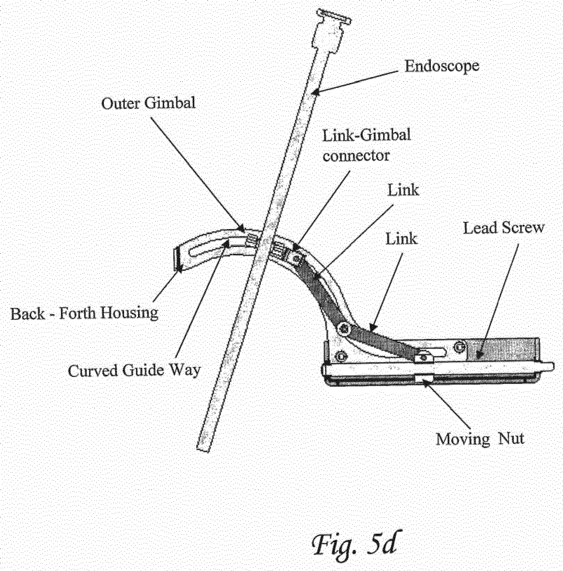

Reference is made now to figure 5d, illustrating a cut view of the first part. Rotating the lead screw cause the linear movement of the moving nut. In the case where the nut moves forward, it pushes the link that is connected to. The link movement is guided by tiny wheels that are placed in the curved guide way. The movement of the link is passed to the outer gimbal directly via a connector or like in fig. 5c via another link. There is no principal limit to number of links except the physical dimensions of the mechanism.

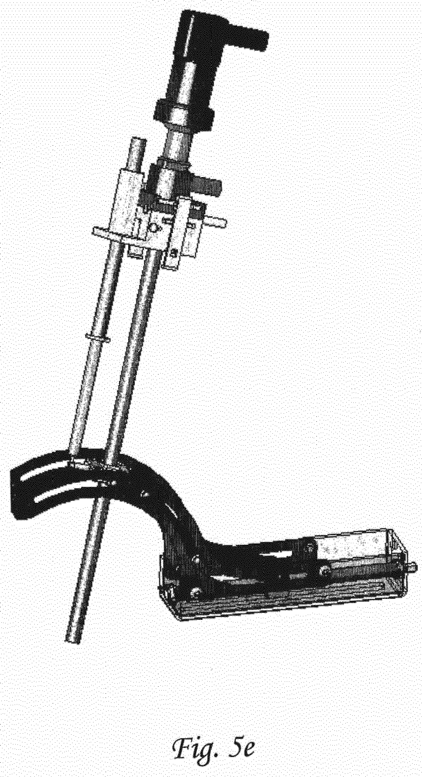

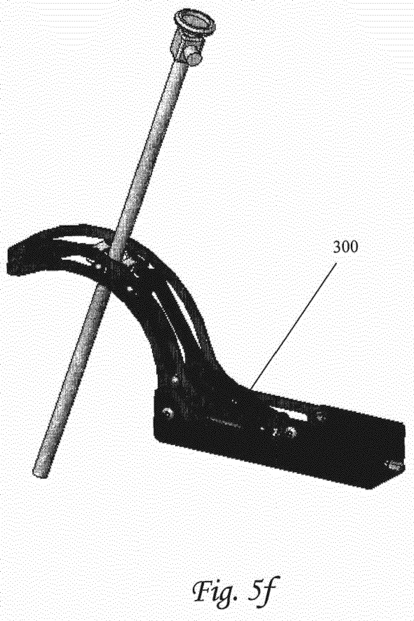

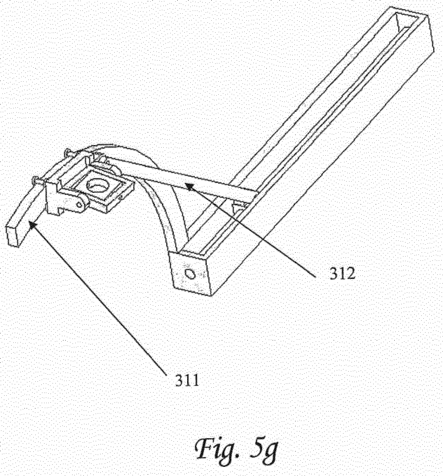

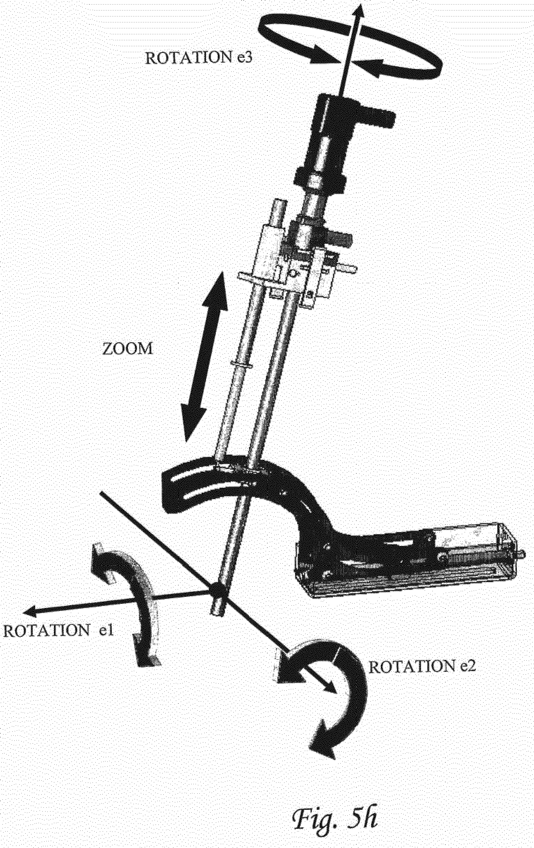

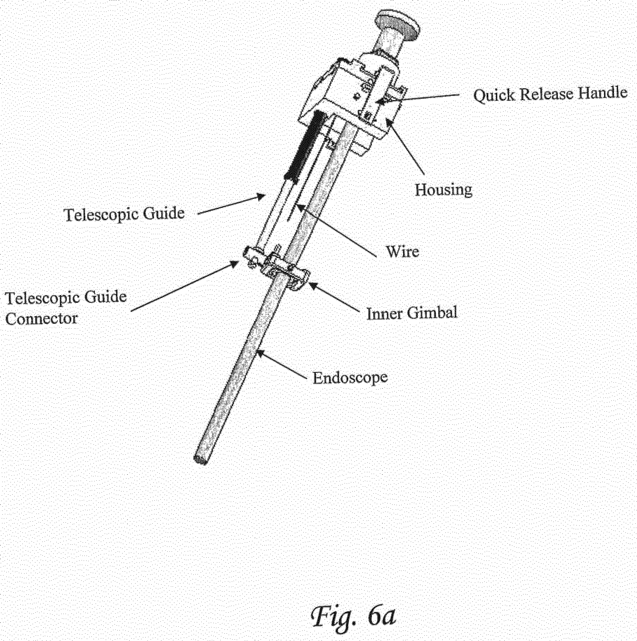

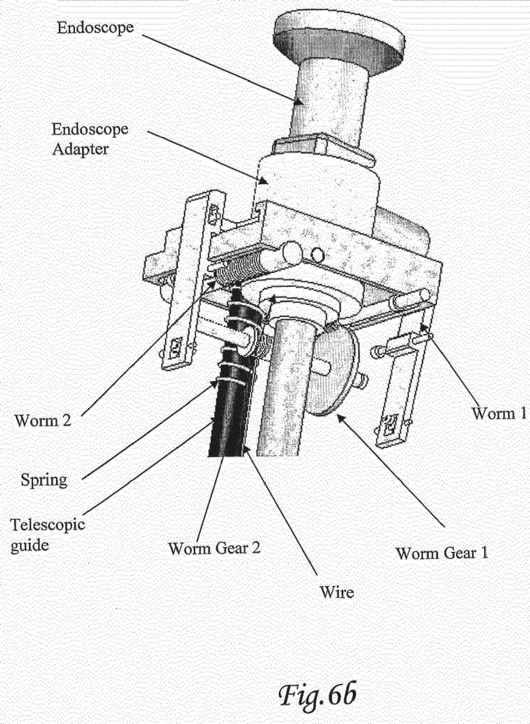

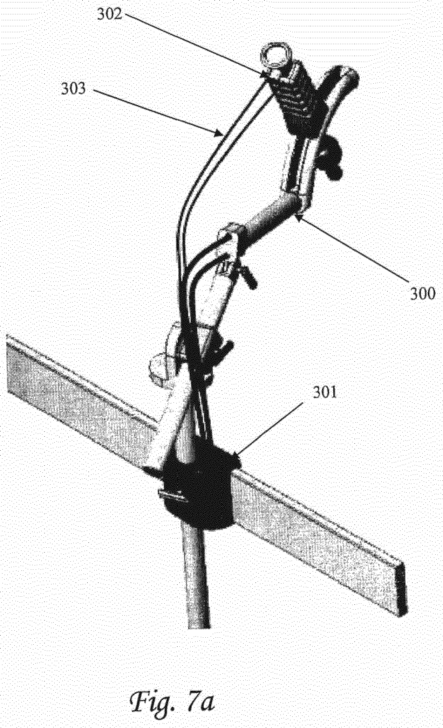

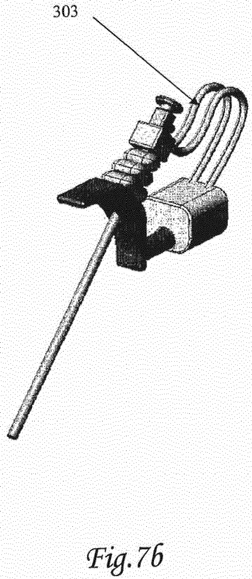

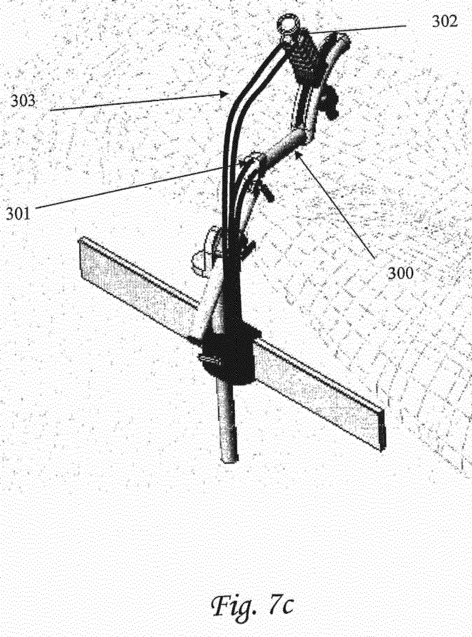

Reference is made now to figures 5e, 5f, illustrating different view of the entire mechanism.
Reference is made now to figure 5g illustrating another realization of the mechanism using only one curved guide way 311, and a single chain of links 312. This structure has some more advantages: the whole mechanism is thinner and allows a faster connection and disconnection of the endoscope from the mechanism, for example in a case when cleaning of the endoscope lens is needed.
Reference is made now to figure 5h illustrating four degrees of freedom of the mechanism.
Reference is made now to figure 6a, illustrating the second part of the mechanism. The second part includes a tiny mechanism enabling the movement of the endoscope in two other manners: a zoom movement, where the endoscope moves along its long axis and a rotation movement of the endoscope around its long axis.
Reference is made now to figure 6b, illustrating the telescopic guide mechanism.
In order to produce the zoom movement, the mechanism consists of a wire and spring. The spring moves the endoscope in the upper direction and the wire which is wrapped on the drum pull the endoscope downward. The exact position is determined by the length of the wire and the force applied by the spring causing the wire to be stretched at any desired position. In order to wrap the wire against the force of the spring, a worm gear transmission is used to convert the rotation of the flexible shafts into a change of the wire length. The telescopic guide serves the two purpose of preventing a possible rotation of the housing of the mechanism and centering the outer spring.
Reference is made now to figures 7a, b, c illustrating an example of a camera holder mechanism for laparoscopic surgery. The camera holder comprises a motor house 301 and a zoom and roll mechanism 302, a sliding DF, a rotating DF, arms for polling the slider (300) and tubes with flexible wire that transmit the rotation moment to the component of the zoom mechanism (303).

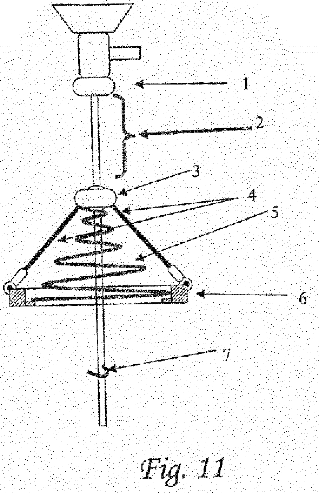

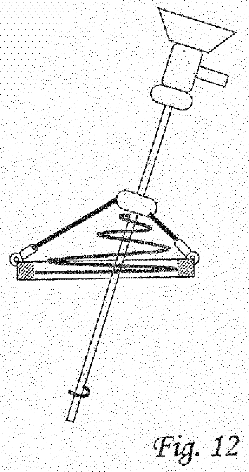

The present invention generally relates to means controlling an endoscope system for laparoscopic surgery, in which the endoscope is inserted through a small incision in the abdomen or chest as illustrated in figure 8.
It is therefore one object of the present invention to present a novel means for controlling the spatial position of endoscope tube in laparoscopic surgery. The present device is cheap, easily install and disassemble, comfortable to use, not limiting the dexterity of the surgeon and having small physical dimension.
The small size of present invention is achieved by applying the following steps:
1. separating the moving parts from the motors and transmitting the motor power by cable means;
2. applying a linear zoom mechanism, allowing a full range zoom action, independent of other moving parts in the mechanism, e.g. not like other robots that achieve the linear zoom action, by a combined movement of the robot arms;
3. obtaining a rotational mechanism that rotates the endoscope about its long axis, independently of other moving parts of the mechanism, e.g., not like other robots that does not as the ability to compensate un wanted rotational movements, or by a combined movement of the robot arms that produce big movements in order to achieve small rotations.
Reference is made now to figure 11, presenting a schematic illustration of the entire device according to one embodiment of the present invention. Said device inter alia comprising a grasp ring (1); Zoom mechanism (2); Orientation ring (3); Cables L₁ and L₂ (4); a spring (5); and a basis ring (6). (7) Described the pinhole in the operated body. When the lengths of L₁ and L₂ are changed in conjunction with the spring resistance, the orientation ring is moved relatively to the basis ring and get to an equilibrium point as illustrated in fig.12. Although a zoom action can be obtained by coordinated shortening of the cables L₁, L₂, L₃, the mechanism includes an additional zoom option that acts independently of cable lengths L₁, L₂, L₃.

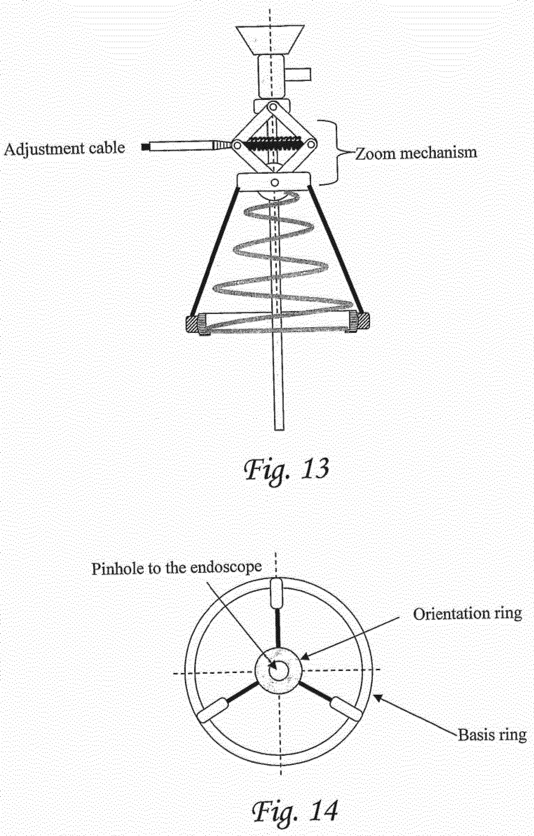

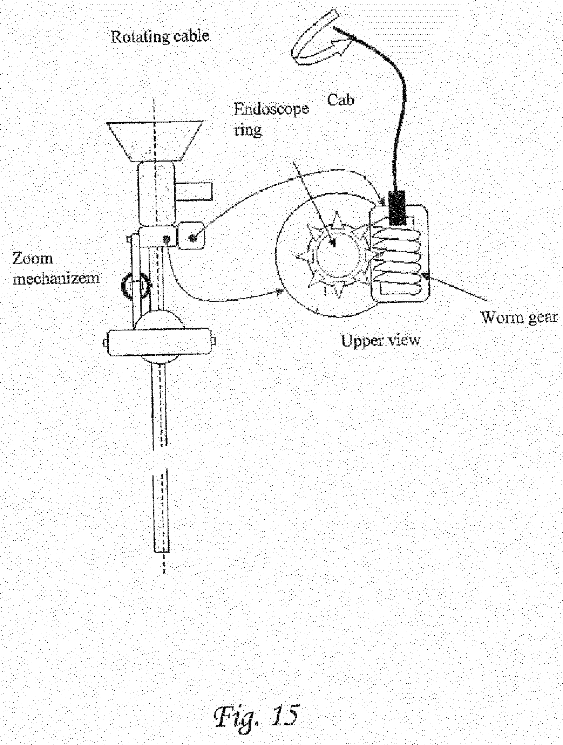

The zoom action is the endoscope movement in front and backward without changing the orientation. The zoom mechanism is schematically described in figure 13. The different lengths of the three cables, when tensing fix the place of the orientation ring as illustrated in figure 14. The mechanism controlling the cables length allows a shifting of the orientation cable and an inclination of the tube to a wanted angle. The endoscope has to rotate around its length axis when the surgeon operates without changing the orientation. Reference is made now to figure 15 presenting a schematic description of the rotation mechanism.

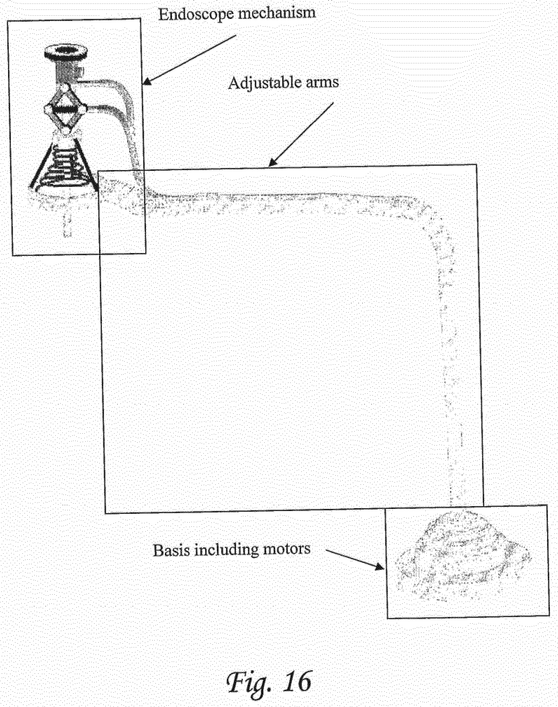

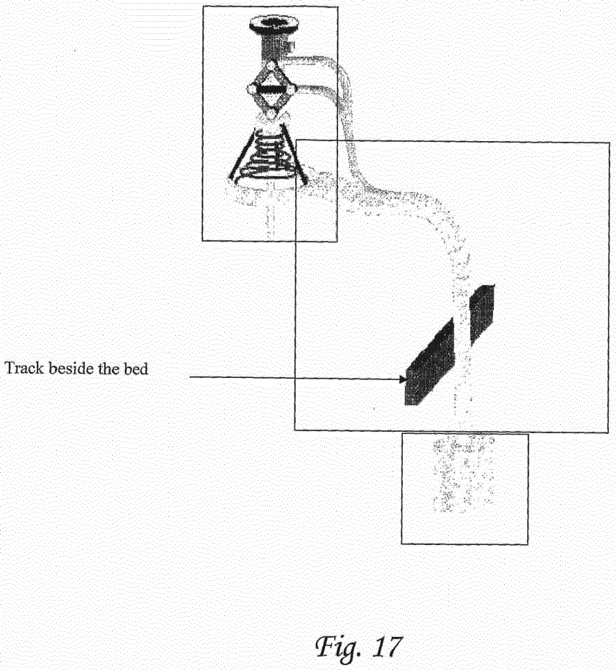

Reference is made now to the portable feature of the mechanism as described in figure 16. The mechanism can be placed beside a bed, as described in figure 17.

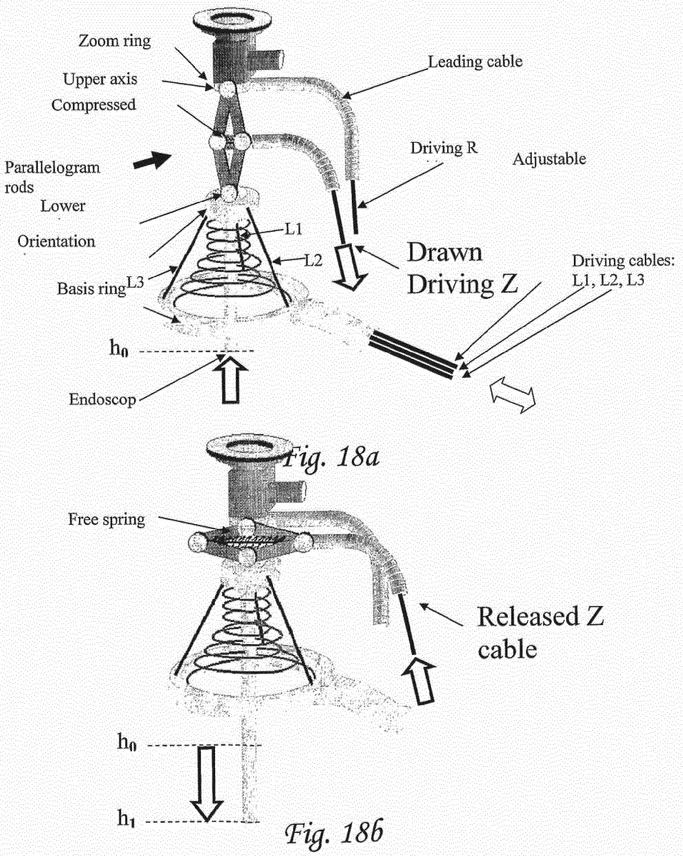

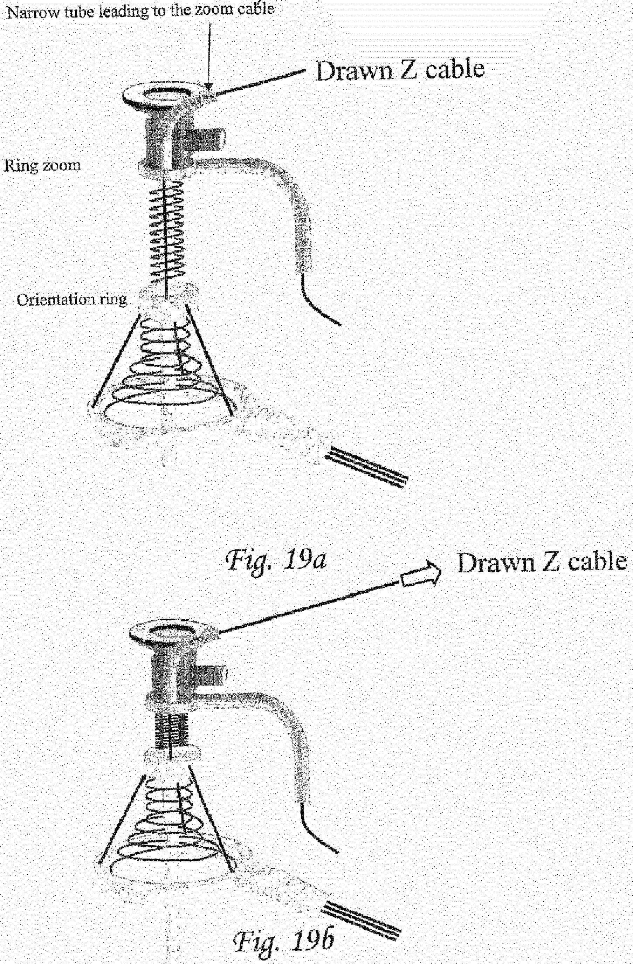

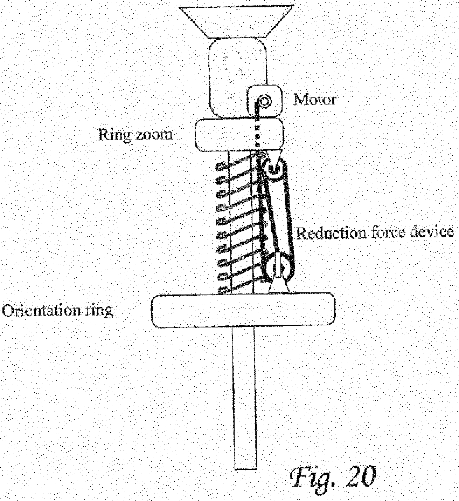

Three options are proposed for the zoom mechanism: one with parallelogram rods, the second with a ring zoom and the third with a reduction force device as described in figures 18a, 18b, 19a, 19b, 20.

A tiny motor wraps the Z cable. The stake system allows on one hand a reduction of the required force that compress the spring , and one the other hand an augmentation of the zoom movement sensibility. In order to obtain a small zoom movement, many windings are required.

When the Z cable is pulling, the distance between the zoom ring and the orientation one is reduced. In this manner the zoom movement is produced. The release and the tense of the Z cable allow continually the deep fixing of the zoom.

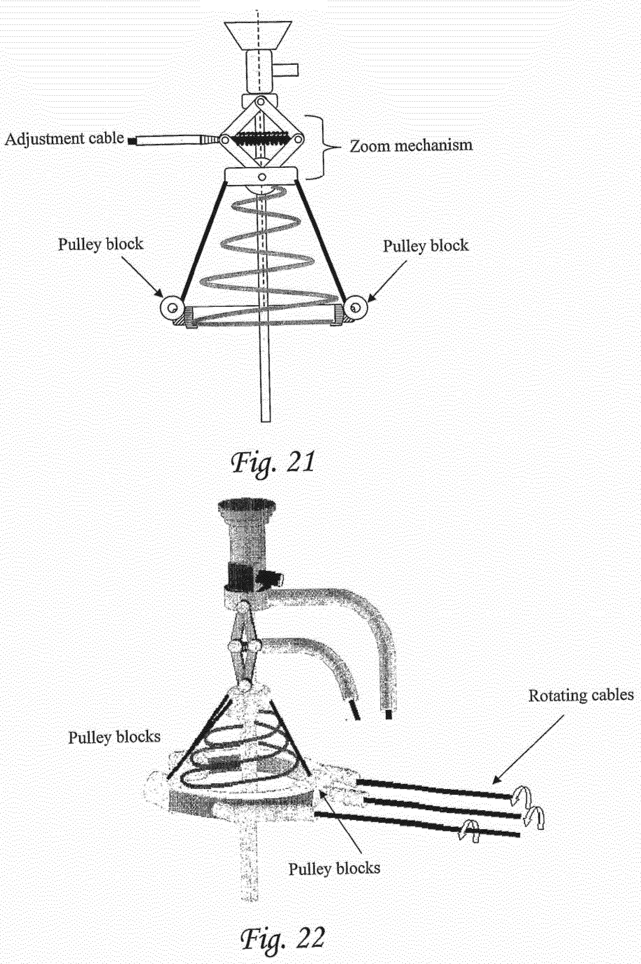

Another alternative is to base the cable length change on pulley blocks motion. The pulley blocks are located on the endoscope motion mechanism .Reference is made now the figure 21, presenting a schematic section view of this. In figure 22, a three-dimension description is proposed.

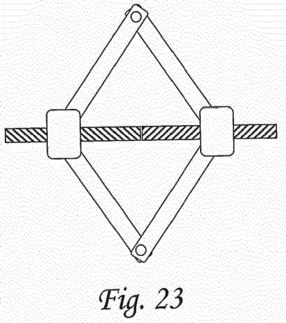

The realization of the zoom mechanism can be done by rotating cable which turns a central screw with joins in different directions as illustrated in figure 23.

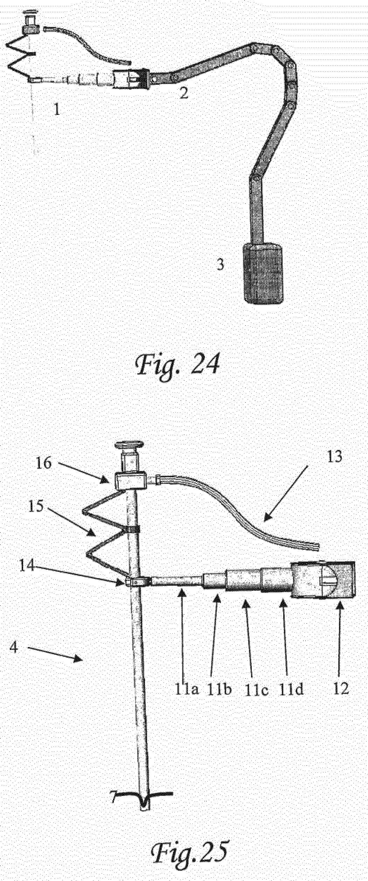

Reference is made now to any of figures 10 and 24, presenting a schematic and illustrated drawing of the entire device according to one embodiment of the present invention. The device comprises inter alia a manipulating endoscope mechanism (1); a force carriage system (2); and a manipulating system actuator (3).

Reference is made now to figure 25, presenting a schematic illustration of the manipulating endoscope mechanism (1). The mechanism comprises inter alia a rotating link (12); linear links (11 a, b, c, d); gimbals ring mechanism (14); zoom leading bars (15); zoom and rotation endoscope mechanism (16); cables tubes (13). The pinhole in the operated body is illustrated by 70, where the endoscope (4) passes through into the abdomen cavity.

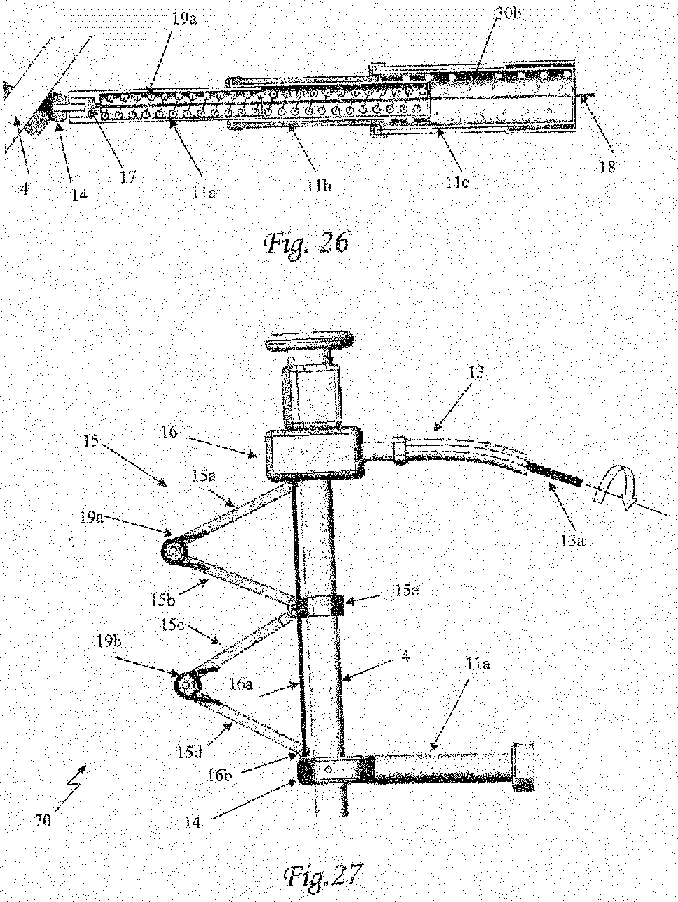

Reference is made now to figure 26, presenting a schematic cut view along the sliding links 11a,b,c. The cable head 17 is mounted in a hole at the head of link 11a. When the links 11a is pulled by cable 18 it slide into links lib against the pushing force of springs 19 a, b and therefore the distance between the center of the gimbals 14 and the center of the rotating link 12 becomes shorter. When the cable 18 is released, springs 19 push links 1 la out of links 1 lb and 1 lb out of links I ie, and the distance between the center of the gimbals 14 and the center of the rotating link 12 becomes longer, in both cases the gimbals is moved relatively to the pinhole and changes the orientation of the endoscope. When the cable does not move, equilibrium is kept at every point by the pushing forces of the springs that tend to push the link outward, and the cable tension. The zoom action is essential in laparoscopic surgery. Changing the zoom enables the surgeon to see important details of the operated organs e.g. "zoom in", and to examine the general situation of the operation status when moving the endoscope away from the scenery e.g. "zoom out". Another important feature is the ability to make a zoom movement while keeping the center of the picture without movement. This could be achieved if the zoom movement is done without changing the endoscope orientation.

Reference is made now to figure 27, presenting the zoom mechanism, fulfilling the needs mentioned above. The zoom action is the endoscope movement into (zoom in) and out of the abdomen cavity (zoom out), without changing the endoscope orientation. The "zoom in" action is obtained by shortening cable 16a, and the "zoom out" action is obtained by extending the length of cable 16a. The springs 19a and 19b tend to increase the angle between the pair of links 15a 15b, and the pair 15c 15d and so to produce the "zoom out" movement. The length of cable 16a determines the amount of the "zoom in". When the endoscope does not move, there is equilibrium between the springs force and the cable tension. Box 16 contains two separate mechanisms that control the linear movement of the endoscope e.g. "zoom in", "zoom out" and the rotation angle of the endoscope along the long axis.

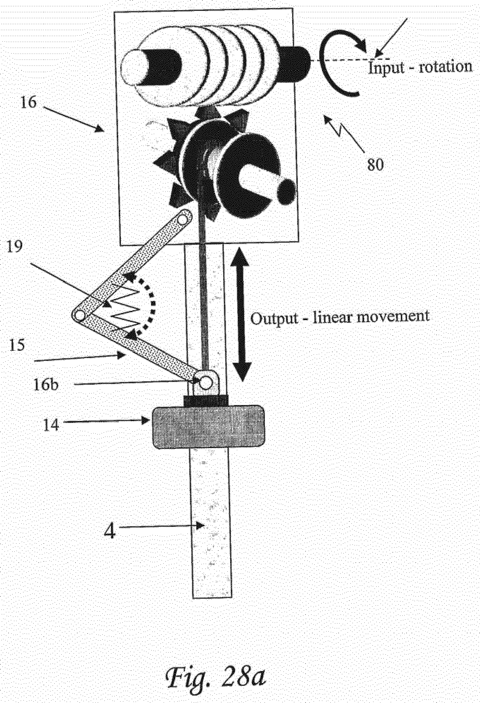

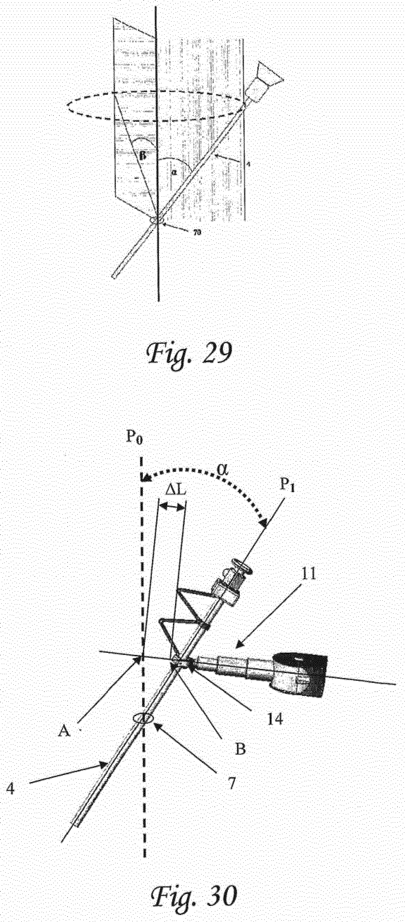

Reference is made now to figure 28a, presenting schematically a principle mechanism that controls the linear movement of the endoscope e.g. "zoom in", "zoom out" by changing the length of cable 16a length. The ability to rotate about the endoscope along its long axis is essential in laparoscopic surgery. While rotating the endoscope through the insertion point (7) in order to change the endoscope orientation e.g. combination of angle α and angle β shown at figure 29, a component of the angular change may be not along the long axis of the endoscope. This angular component may cause undesirable rotation of the endoscope, which in result, cause annoying rotating movement of the picture as viewed on the surgeon's video screen. In a traditional laparoscopic operation, the person that holds the endoscope, intuitively, makes the needed changes to keep the operation scenery without uii desirable rotation e.g., keeping the moving picture parallel to itself at all time.

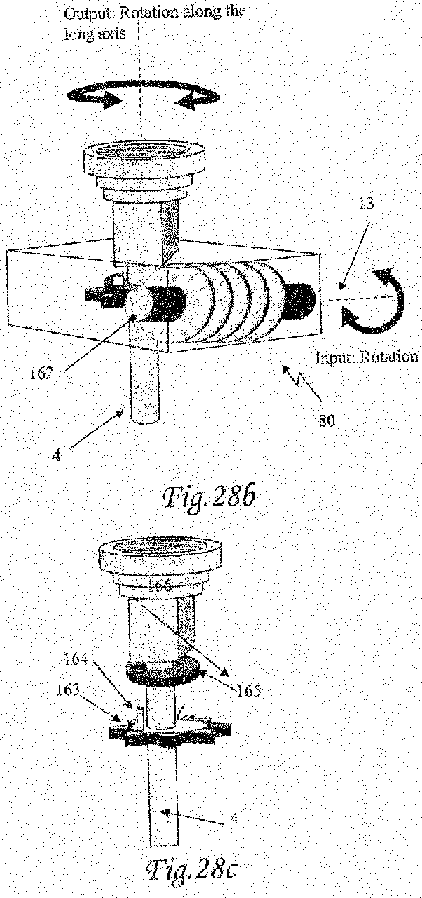

Reference is made now to figure 28b and figure 28c, presenting the rotation mechanism, filling the needs mentioned above, and also allowing fast removal of the endoscope in order to clean its lens. The cog-wheel 163 allows the endoscope rod 4 to cross through its center and to make roll and sliding movements. The peg 164 arises from cog-wheel 163 upper surface. A disk 165 is tightened to the endoscope rod 4. While assembling the endoscope the upper wall of the box 16 is opened and the endoscope is entered through cog-wheel 163 center, into a hole in the lower wall and through ring e.g. gimbals 14 until the peg 164 is threaded into aperture 166 of disk 165. Then the upper wall of box 16 is closed, keeping the endoscope from moving out of box 16, to ensure coupling between the endoscope and the entire zoom mechanism. The rotation of the endoscope along is achieved by rotating the screw 162 that moves cog-wheel 163 and the endoscope 4 via coupled disk 165. The source of the movement of screw 162 can be a rotating cable transmitting the rotation movement from "remote" motor or small motor placed in or near box 16. When needed, the mechanism described above allows quick disassembling of the endoscope out of the zoom mechanism without changing any degree of freedom of its spatial position. This property is important because the surgeon does not have to deal with re-positioning of the system. This property is achieved because the endoscope 4 does not have any role in keeping the position of the entire zoom mechanism. The equilibrium between links 15 springs 19 and cable 16a maintain depth of the zoom and constrain of relation between the peg 164 and hole 166 keeps the angle of rotation. When the endoscope is assembled again, the endoscope retrieves its original spatial position. While executing the operation the surgeon must be able to move the endoscope to any desired orientation. The envelope of the endoscope range of movement is shown in figure 29.

Reference is made now to figure 30, presenting schematically the way the mechanism acts to controls one angle of the endoscope by changing the total length of the telescopic arm. Figure 30 shows the angular movement of endoscope 4 that was at starting position PO e.g. α=0. Activating the sliding mechanism causes the movement of gimbals rings 14 from point A to point B causing endoscope 4 to rotate about the insertion point 70, to a desired position PI . While the combined shortening of links 11 a, b, c, the distance between gimbals 14 and the insertion point 70 changes, causing an undesired zoom movement. The distance of this movement can be calculated and compensated by a controlled zoom motion.

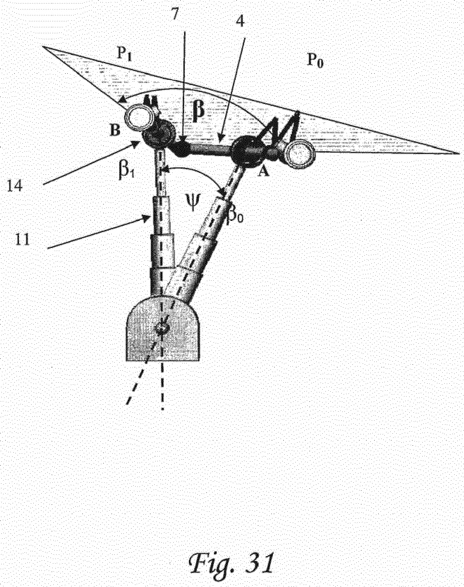

Reference is made now to figure 31, schematically presenting the way the mechanism acts to controls another angle e.g. β of the endoscope by rotating telescopic arm. Figure 31 shows the angular movement of endoscope 4 that was at starting position P0. Activating the rotating mechanism causes the movement of gimbals rings 14 in a radial movement, from point A to point B e.g. angle ψ, causing endoscope 4 to rotate about the insertion point 70, by angle β, to a desired position PI. While the rotating of arm 11, the distance between gimbals 14 and the insertion point 70 changes, causing an undesired zoom movement. The distance of this movement can be calculated and compensated by a controlled zoom motion. The combination of the two independent movements of the mechanism arm enables the surgeon to move the endoscope to any orientation, and reach any desired point within the working envelope.

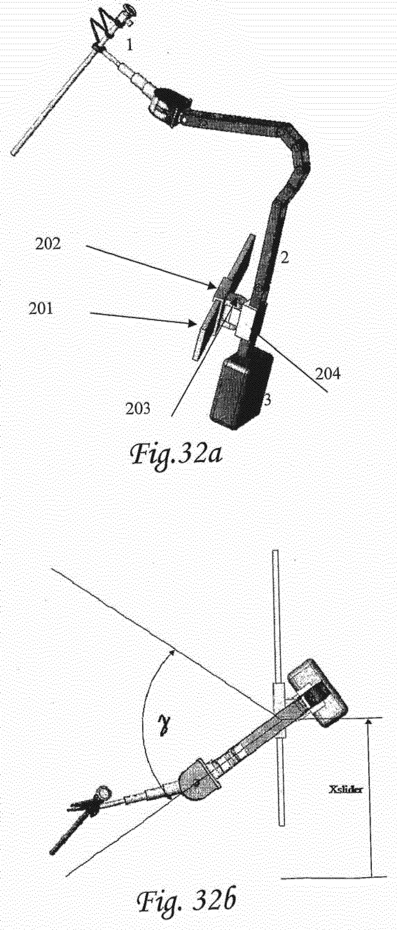

Reference is made now to the portable feature of the mechanism as described in figure 32a. The mechanism is placed beside a bed, on track 201, and can be placed at any point along track 201 by moving slider 202; in order to achieve the necessary position the surgeon can also rotate the system around pivot 203 and to change the height by sliding the system along house 204. Figure 32b shows schematically from upper view, the position abilities of the system: the rotation angle γ, and the horizontal position X slider.

## Claims

1. A device comprising:
a. an automated endoscope assistant (19) for moving an endoscope (21);
b. at least one wireless transmitter (12a, 12b, 12c) with at least one operating key adapted to transmit a code upon depression of said at least one operating, key;
c. at least one wireless receiver (11) adapted to receive the code of the at least one wireless transmitter;
d. at least one video screen (30);
e. at least one conventional laparoscopy computerized system (15) connected to the at least one wireless receiver (11), the automated endoscope assistant (19) and the at least one video screen (30), and loaded with conventional surgical instrument spatial location software, and conventional automated assistant maneuvering software;
f. software loaded onto the conventional laparoscopy computerized system (15) that is configured to enable, a visual selection of an instrument area on the video screen (30) in response to the depression of the at least one operating, key on the at least one wireless transmitter (12a, 12b, 12c) as well as an interface with the conventional automated assistant maneuvering software so as to achieve movement of the endoscope (21), said endoscope thus providing a view on the video screen (30) that is focused on the selected instrument area.

2. The device of claim 1 wherein said at least one wireless transmitter (12a) is freestanding.

3. The device of claim 1 wherein the at least one wireless transmitter (12b, 12c) is fitted to at least one surgical instrument (17b, 17c).

4. The device of any one of claims 1 to 3, wherein the software loaded onto the conventional laparoscopy computerized system (15) is configured for producing a temporary onscreen graphic symbol (35b, 35c) on a random surgical instrument (37b, 37c), so that upon repeating the depression of the at least one operating key the graphic symbol (35b, 35c) is shifted from onscreen depiction of one surgical instrument to another until a desired surgical instrument (37b, 37c) is selected, achieving a selection input for the computerized system (15) and directing it to maneuver the endoscope (21) providing the view focused on the selected instrument area.

5. The device of claim 4 wherein the software loaded onto the conventional laparoscopy computerized system (15) is configured such that the graphic symbol (35b, 35c) appears continuously.

6. The device of one of claims 4 and 5 wherein the software loaded onto the conventional laparoscopy computerized system (15) is configured such that an alternate key depression instructs the computerized system (15) to continuously direct the endoscope (21) to follow the selected instrument (37b, 37c).

7. The device of any one of claims 1 to 6 wherein the software loaded onto the conventional laparoscopy computerized system (15) is configured such that additional keys on the at least one wireless transmitter (12b, 12c) can be programmed in order to easily reproduce previously selected views required repeatedly.

8. The device of claim 7 wherein the software loaded onto the conventional laparoscopy computerized system (15) is configured such that depression of the additional keys on the at least one wireless transmitter (12b, 12c) produces a menu of views continuously stored in a database of the computerized system (15).

9. The device according to any one of claims 1 to 8, wherein the software loaded onto the conventional laparoscopy computerized system (15) is configured such that prior to a procedure the at least one operating key of the at least one wireless transmitter (12a, 12b, 12c) can be depressed for matching the transmitted code with surgical instrument characteristics previously stored in a database of the computerized system (15).

10. The device of claim 9 wherein the software loaded onto the conventional laparoscopy computerized system (15) is configured such that a selection of a surgical instrument (37b, 37c) is signified by a temporary onscreen graphic symbol (35b, 35c) presented upon an onscreen depiction of the surgical instrument (37b, 37c).

11. The device of claim 9 wherein the software loaded onto the conventional laparoscopy computerized system (15) is configured such that a selection of a surgical instrument (37b, 37c) is signified by a continuous onscreen graphic symbol (35b, 35c) presented upon an onscreen depiction of the surgical instrument (37b, 37c).

12. The device of claim 9 wherein the software loaded onto the conventional laparoscopy computerized system (15) is configured such that a selection of a surgical instrument (37b, 37c) is further confirmed by depressing the at least one operating key on the at least one wireless transmitter (12b, 12c) authorizing the computerized system (15) to maneuver the endoscope by means of an automated assistant arm (19).

## Patentansprüche

1. Vorrichtung, umfassend:
a. einen automatisierten Endoskopassistenten (19) zum Bewegten eines Endoskops (21);
b. mindestens einen drahtlosen Sender (12a, 12b, 12c) mit mindestens einer Bedientaste, der dazu eingerichtet ist, einen Code zu senden, sobald die mindestens eine Bedientaste gedrückt wird;
c. mindestens einen drahtlosen Empfänger (11), der zum Empfangen des Codes von dem mindestens einen drahtlosen Sender eingerichtet ist;
d. mindestens einen Videobildschirm (30);
e. mindestens ein herkömmliches computergestütztes Laparoskopiesystem (15), das mit dem mindestens einen drahtlosen Empfänger (11), dem automatisierten Endoskopassistenten (19) und dem mindestens einen Videobildschirm (30) verbunden und mit herkömmlicher Software zur räumlichen Ortung chirurgischer Instrumente und herkömmlicher Software zum Manövrieren automatisierter Assistenten ausgestattet ist;
f. auf das herkömmliche computergestützte Laparoskopiesystem (15) geladene Software, die dazu konfiguriert ist, eine visuelle Auswahl eines Instrumentenbereichs auf dem Videobildschirm (30) in Reaktion auf das Drücken der mindestens einen Bedientaste an dem mindestens einen drahtlosen Sender (12a, 12b, 12c) zu ermöglichen, sowie eine Schnittstelle mit der herkömmlichen Software zum Manövrieren automatisierter Assistenten, um Belegung des Endoskops (21) zu erzielen, wobei das Endoskop somit auf dem Videobildschirm (30) eine Ansicht bereitstellt, die auf den ausgewählte Instrumentenbereich konzentriert ist.

2. Vorrichtung nach Anspruch 1, wobei der mindestens eine drahtlose Sender (12a) freistehend ist.

3. Vorrichtung nach Anspruch 1, wobei der mindestens eine drahtlose Sender (12b, 12c) an mindestens einem chirurgischen Instrument (17b, 17c) angebracht ist.

4. Vorrichtung nach einem der Ansprüche 1 bis 3, wobei die auf das herkömmliche computergestützte Laparoskopiesystem (15) geladene Software dazu konfiguriert ist, ein zeitweiliges graphisches Bildschirmsymbol (35b, 35c) auf einem beliebigen chirurgischen Instrument (37b, 37c) zu produzieren, sodass beim Wiederholen des Drückens der mindestens einen Bedientaste das grafische Symbol (35b, 35c) von der Bildschirmdarstellung eines chirurgischen Instruments zu einem anderen verschoben wird, bis ein gewünschte chirurgisches Instrument (37b, 37c) ausgewählt ist, wodurch eine Auswahleingabe für das computergestützte System (15) erzielt und es gelenkt wird, sodass es das Endoskop (21) manövriert, das die auf den ausgewählte Instrumentenbereich konzentrierte Ansicht bereitstellt.

5. Vorrichtung nach Anspruch 4, wobei die auf das herkömmliche computergestützte Laparoskopiesystem (15) geladene Software so konfiguriert ist, dass das grafische Symbol (35b, 35c) kontinuierlich erscheint.

6. Vorrichtung nach einem der Ansprüche 4 und 5, wobei die auf das herkömmliche computergestützte Laparoskopiesystem (15) geladene Software so konfiguriert ist, dass ein anderer Tastendruck das computergestützte System (15) anweist das Endoskop (21) kontinuierlich zu lenken, sodass es dem ausgewählte Instrument (37b, 37c) folgt.

7. Vorrichtung nach einem der Ansprüche 1 bis 6, wobei die auf das herkömmliche computergestützte Laparoskopiesystem (15) geladene Software so konfiguriert ist, dass zusätzliche Tasten an dem mindestens einen drahtlosen Sender (12b, 12c) programmiert werden können, um zuvor ausgewählt Ansichten, die wiederholt benötigt werden, auf einfache Weise zu reproduzieren.

8. Vorrichtung nach Anspruch 7, wobei die auf das herkömmliche computergestützte Laparoskopiesystem (15) geladene Software so konfiguriert ist, dass das Drücken der zusätzlichen Tasten an dem einen drahtlosen Sender (12b, 12c) ein Menü von Ansichten produziert, die kontinuierlich in einer Datenbank des computergestützten Systems (15) gespeichert sind.

9. Vorrichtung nach einem der Ansprüche 1 bis 8, wobei die auf das herkömmliche computergestützte Laparoskopiesystem (15) geladene Software so konfiguriert ist, dass vor einem Eingriff die mindestens eine Bedientaste des mindestens einen drahtlosen Senders (12a, 12b, 12c) gedrückt werden kann, um den gesendeten Code mit zuvor in einer Datenbank des computergestützten Systems (15) gespeicherten Merkmalen chirurgischer Instrumente abzugleichen.

10. Vorrichtung nach Anspruch 9, wobei die auf das herkömmliche computergestützte Laparoskopiesystem (15) geladene Software so konfiguriert ist, dass eine Auswahl eines chirurgischen Instruments (37b, 37c) durch ein zeitweiliges grafisches Bildschirmsymbol (35b, 35c) signalisiert wird, das bei einer Bildschirmdarstellung des chirurgischen Instruments (37b, 37c) gezeigt wird.

11. Vorrichtung nach Anspruch 9, wobei die auf das herkömmliche computergestützte Laparoskopiesystem (15) geladene Software so konfiguriert ist, dass eine Auswahl eines chirurgischen Instruments (37b, 37c) durch ein kontinuierliches grafisches Bildschirmsymbol (35b, 35c) signalisiert wird, das bei einer Bildschirmdarstellung des chirurgischen Instruments (37b, 37c) gezeigt wird.

12. Vorrichtung nach Anspruch 9, wobei die auf das herkömmliche computergestützte Laparoskopiesystem (15) geladene Software so konfiguriert ist, dass eine Auswahl eines chirurgischen Instruments (37b, 37c) weiter durch Drücken der mindestens einen Bedientaste an dem mindestens einen drahtlosen Sender (12b, 12c) bestätigt wird, wodurch das computergestützte System (15) autorisiert wird, das Endoskop mittels eines automatisierten Assistentenarms (19) zu manövrieren.

## Revendications

1. Dispositif comprenant :
a. un assistant endoscope automatisé (19) pour le déplacement d'un endoscope (21) ;
b. au moins un émetteur sans fil (12a, 12b, 12c) comprenant au moins une touche d'utilisation conçue pour émettre un code lorsqu'on appuie sur ladite au moins une touche d'utilisation ;
c. au moins un récepteur sans fil (11) conçu pour recevoir le code dudit au moins un émetteur sans fil ;
d. au moins un écran vidéo (30) ;
e. au moins un système informatisé conventionnel de laparoscopie (15) connecté audit au moins un récepteur sans fil (11), audit assistant endoscope automatisé (19) et audit au moins un écran vidéo (30), et chargé avec un logiciel conventionnel de localisation dans l'espace d'un instrument chirurgical et avec un logiciel conventionnel de manoeuvre d'un assistant automatisé ;
f. un logiciel chargé sur le système informatisé conventionnel de laparoscopie (15), qui est configuré pour permettre une sélection visuelle d'une zone d'instrument sur l'écran vidéo (30) en réponse au fait d'appuyer sur ladite au moins une touche d'utilisation sur ledit au moins un émetteur sans fil (12a, 12b, 12c) ainsi qu'une interface avec le logiciel conventionnel de manoeuvre de l'assistant automatisé de façon à mettre en oeuvre un mouvement de l'endoscope (21), ledit endoscope procurant ainsi une vue sur l'écran vidéo (30) qui est focalisée sur la zone d'instrument sélectionnée.

2. Dispositif selon la revendication 1, dans lequel ledit au moins un émetteur sans fil (12a) est autonome.

3. Dispositif selon la revendication 1, dans lequel ledit au moins un émetteur sans fil (12b, 12c) est adapté à au moins un instrument chirurgical (17b, 17c).

4. Dispositif selon l'une quelconque des revendications 1 à 3, dans lequel le logiciel chargé sur le système informatisé conventionnel de laparoscopie (15) est configuré pour produire un symbole graphique temporaire sur écran (35b, 35c) sur un instrument chirurgical aléatoire (37b, 37c) d'une manière telle que lorsqu'on appuie à nouveau sur ladite au moins une touche d'utilisation, le symbole graphique (35b, 35c) est décalé de la reproduction sur écran d'un instrument chirurgical à celle d'un autre jusqu'à ce qu'un instrument chirurgical désiré (37b, 37c) ait été sélectionné, réaliser une entrée de sélection pour le système informatisé (15) et le diriger pour manoeuvrer l'endoscope (21) procurant la vue focalisée sur la zone d'instrument sélectionnée.

5. Dispositif selon la revendication 4, dans lequel le logiciel chargé sur le système informatisé conventionnel de laparoscopie (15) est configuré d'une manière telle que le symbole graphique (35b, 35c) apparaît en continu.

6. Dispositif selon une des revendications 4 ou 5, dans lequel le logiciel chargé sur le système informatisé conventionnel de laparoscopie (15) est configuré d'une manière telle qu'un appui alterné sur la touche donne instruction au système informatisé (15) de diriger en continu l'endoscope (21) pour suivre l'instrument sélectionné (37b, 37c).

7. Dispositif selon l'une quelconque des revendications 1 à 6, dans lequel le logiciel chargé sur le système informatisé conventionnel de laparoscopie (15) est configuré d'une manière telle que des touches supplémentaires sur ledit au moins un émetteur sans fil (12b, 12c) peuvent être programmées dans le but de reproduire aisément des vues préalablement sélectionnées requises de manière répétée.

8. Dispositif selon la revendication 7, dans lequel le logiciel chargé sur le système informatisé conventionnel de laparoscopie (15) est configuré d'une manière telle que le fait d'appuyer sur les touches supplémentaires sur ledit au moins un émetteur sans fil (12b, 12c) produit un menu de vues stockées en continu dans une base de données du système informatisé (15).

9. Dispositif selon l'une quelconque des revendications 1 à 8, dans lequel le logiciel chargé sur le système informatisé conventionnel de laparoscopie (15) est configuré d'une manière telle qu'avant une opération, on peut appuyer sur ladite au moins une touche d'utilisation dudit au moins un émetteur sans fil (12a, 12b, 12c) pour la mise en correspondance du code transmis avec des caractéristiques d'instruments chirurgicaux préalablement stockées dans une base de données du système informatisé (15).

10. Dispositif selon la revendication 9, dans lequel le logiciel chargé sur le système informatisé conventionnel de laparoscopie (15) est configuré d'une manière telle qu'une sélection d'un instrument chirurgical (37b, 37c) est signifiée par un symbole graphique temporaire sur écran (35b, 35c) présenté lors d'une représentation sur écran de l'instrument chirurgical (37b, 37c).

11. Dispositif selon la revendication 9, dans lequel le logiciel chargé sur le système informatisé conventionnel de laparoscopie (15) est configuré d'une manière telle qu'une sélection d'un instrument chirurgical (37b, 37c) est signifiée par un symbole graphique sur écran en continu (35b, 35c) présenté lors d'une représentation sur écran de l'instrument chirurgical (37b, 37c).

12. Dispositif selon la revendication 9, dans lequel le logiciel chargé sur le système informatisé conventionnel de laparoscopie (15) est configuré d'une manière telle qu'une sélection d'un instrument chirurgical (37b, 37c) est en outre confirmée par le fait d'appuyer sur ladite au moins une touche d'utilisation dudit au moins un émetteur sans fil (12b, 12c) autorisant le système informatisé (15) à manoeuvrer l'endoscope au moyen d'un bras assistant automatisé (19).
